(19) Europäisches Patentamt
European Patent Office
Office européen des brevets

(11) **EP 3 977 925 A1**

(12) **EUROPEAN PATENT APPLICATION**
published in accordance with Art. 153(4) EPC

(43) Date of publication:
**06.04.2022 Bulletin 2022/14**

(21) Application number: **20815299.1**

(22) Date of filing: **28.05.2020**

(51) International Patent Classification (IPC):
***A61B 5/0408*** (2006.01)

(52) Cooperative Patent Classification (CPC):
**A61B 5/296; A61B 5/265; A61B 5/268; A61B 5/27;**
**A61N 1/0452; A61N 1/36003;** A61B 5/224;
A61B 5/256; A61B 5/28; A61B 5/291; A61B 5/4836;
A61B 5/6824; A61B 5/6828; A61B 2505/09

(86) International application number:
**PCT/JP2020/021068**

(87) International publication number:
**WO 2020/241733 (03.12.2020 Gazette 2020/49)**

(84) Designated Contracting States:
**AL AT BE BG CH CY CZ DE DK EE ES FI FR GB**
**GR HR HU IE IS IT LI LT LU LV MC MK MT NL NO**
**PL PT RO RS SE SI SK SM TR**
Designated Extension States:
**BA ME**
Designated Validation States:
**KH MA MD TN**

(30) Priority: **31.05.2019 JP 2019102379**
**29.08.2019 JP 2019156344**

(71) Applicant: **Seiren Co., Ltd.**
**Fukui-shi**
**Fukui 918-8560 (JP)**

(72) Inventors:
• **HAYASHI, Hiroyuki**
 **Fukui-shi, Fukui 918-8560 (JP)**
• **TSUJIMOTO, Kazuhisa**
 **Fukui-shi, Fukui 918-8560 (JP)**

(74) Representative: **Hoffmann Eitle**
**Patent- und Rechtsanwälte PartmbB**
**Arabellastraße 30**
**81925 München (DE)**

(54) **BIOELECTRODE AND BIOELECTRODE-EQUIPPED APPARATUS**

(57) [Problem] Provided are a bioelectrode that can measure a biological signal having few artifacts, that is less likely to cause shortcircuiting with a nearby bioelectrode, and that does not cause discomfort at the time of wearing or measurement of the biological signal; and a bioelectrode-equipped apparatus that is insusceptible to breaking of wiring caused by being pressed at the time of wearing and that allows appropriate measurement and electrical stimulation by applying the bioelectrode to a predetermined portion of a body regardless of the individual variation in the circumference of the portion to which the bioelectrode is attached. [Solution] This bioelectrode is configured by applying a water-absorbing resin to a sheet-like structure including conductive fibers so as to have a moisture retention index of 0.8 or more. This bioelectrode-equipped apparatus comprises a fabric structure having, on a base fabric formed from an elastic fabric, an electrode placement region that includes a wiring formed on a surface of the base fabric, a bioelectrode provided to the terminal end of the wiring, and an insulating layer for covering the wiring, wherein the base fabric has a first extension direction exhibiting relatively low extensibility in the electrode placement region and a second extension direction which is different from the first extension direction and which exhibits higher extensibility than the first extension direction, and the wiring is formed along the first extension direction.

[Fig. 5]

## Description

### Technical Field

The present invention relates to a bioelectrode that has a low skin contact impedance and that can measure a biological signal having few artifacts.

The present invention also relates to a bioelectrode-equipped apparatus that has a wiring which hardly causes disconnection even when a force is applied at the time of wearing and that can apply the bioelectrode to a predetermined position of a body regardless of individual differences in the circumference (a thickness) of the applied part.

### Background Art

People are getting more interested in maintenance and improvement of health recently. For health management, therefore, easier way to measure biological information such as heartbeat signals, electrocardiographic signals, electroencephalographic signals and myoelectric signals, and to use them for health maintenance in everyday life has been developed.

Especially, it has become well-known that, by measurement of biological information such as potential difference between electrodes connected to an arbitrary position on a skin surface, impedance and capacitance, biological activities or the state of a subcutaneous tissue can be observed, and it is desired to use the results for health maintenance and management.

Regarding an electrical stimulation therapy for motor function recovery of paralyzed patients, a low frequency therapy for improvement of stiffness such as a stiff shoulder by electrical stimulation, and EMS (Electrical Muscle Stimulation) wherein a muscle training effect can be obtained by electrical stimulation, it is known that each effect thereof is obtained by applying electrical stimulation to a living body from an electrode in contact with an arbitrary position on the skin surface.

Conventionally, measurement of biological signals or application of electrical stimulation were generally carried out in a rest state such as lying on a bed.

Recently, attempts have been made to apply a measurement result of biological signals during activities to health management. However, there is a problem that the position of the electrode being contacted with the skin is displaced during activities, and as a result, noise (artifact) is included in acquired biological signals.

Patent Document 1 discloses a bioelectrode which has an electrode layer composed of a fiber knitted fabric whose water contact angle on the surface of the fiber knitted fabric is 115° or more. It is said that this bioelectrode enables to acquire a bioelectric signal with high accuracy by efficiently making moisture released from the skin retained between the skin and the surface of the electrode layer.

However, when two or more bioelectrodes are arranged close to each other, moisture retained between the skin and the surface of the electrode layer flows out to cause a short circuit with a nearby bioelectrode. In addition, since moisture is retained between the skin and the surface of the electrode layer, a person to be measured feels discomfort.

A supporter equipped with an electrode on a stretchable fabric has been developed as a body apparatus for measurement of biological signals easily to solve the problem of displacement of a position of the bioelectrode being contacted with the skin during activities. The apparatus can be used easily in a daily life by wrapping around an arm or a leg or by mounting in a cylindrical shape.

However, whereas accuracy of the connected position of electrodes is important for accurate measurement and appropriate electrical stimulation, since there are wide individual differences in a thickness of arms or legs or a body type of the users, it has been needed to prepare a supporter having an adjusted size according to individual differences of the user.

Patent Document 2, on the other hand, provides a biological signal detecting device mounting an annular elastic base material layer, two or more detection means for detecting a biological signal, and an elastic harness for electric conduction connected to the detection means.

However, since the harness for electric conduction is made of a mixture of a conductive yarn and an elastic yarn, a special device is required for manufacture and it is difficult to design the position of electrodes freely.

### Prior Art Documents

### [Patent Document]

Patent Document 1: Jpn. Pat. Laid-Open Publication No.2018-042719

Patent Document 2: Jpn. Pat. Laid-Open Publication No. 2018-078949

**Disclosure of the Invention**

**Problems to be Solved by the Invention**

It is an object of the present invention to provide a bioelectrode that can measure a biological signal having few artifacts without possibility of a short circuit between adjacent bioelectrodes and that does not cause discomfort at the time of wearing or measurement of biological signals.

It is also an object of the present invention to provide a bioelectrode-equipped apparatus that has a wiring which hardly causes disconnection even when a force is applied at the time of wearing and that allows accurate measurement and appropriate electrical stimulation by applying the bioelectrode to a predetermined position of a body regardless of individual differences in the circumference (a thickness) of the applied part.

**Means for Solving the Problems**

As a result of extensive studies, the inventors have found that a bioelectrode having few artifacts with no risk of a short circuit between adjacent bioelectrodes or feeling of discomfort can be provided by allowing moisture to include in the vicinity of the surface of bioelectrode and by preventing more moisture than necessary from being present in a boundary part between the surface of bioelectrode and the skin, and thus the present

invention has completed.

The inventors have also found that a bioelectrode-equipped apparatus in which a wiring is formed along the direction exhibiting low extensibility on a base fabric surface enables to perform accurate measurement and appropriate electrical stimulation while disconnection of a wiring hardly occurs, and thus the present invention has completed.

The present invention provides a bioelectrode and a bioelectrode-equipped apparatus shown as follows:

(1) A bioelectrode comprising a sheet-like structure containing a conductive fiber and a water-absorbing resin applied to said sheet-like structure, which has a moisture retention index of 0.8 or more.

(2) The bioelectrode according to (1), wherein said water-absorbing resin is a cross-linked product of a polyacrylic acid and/or a salt thereof.

(3) The bioelectrode according to (2), wherein said cross-linked product of a polyacrylic acid and/or a salt thereof has a carboxylate equivalent of 150-1500 g/eq.

(4) The bioelectrode according to any one of (1)- (3), wherein said conductive fiber is a synthetic fiber having a metal film on a surface thereof.

(5) The bioelectrode according to any one of (1) - (4), wherein said sheet-like structure is a knitted fabric.

(6) A bioelectrode-equipped apparatus comprising a fabric structure having an electrode placement region which includes:

a base fabric having an elastic property,
a wiring formed on the surface of said base fabric,
a bioelectrode as a biological signal detection means provided at the terminal end of said wiring, and
an insulating layer for covering said wiring, wherein said base fabric has, in the electrode placement region,
a first extension direction which exhibits relatively low extensibility and
a second extension direction which is different from the first extension direction and which exhibits higher extensibility than the first extension direction, and
said wiring is formed along the first extension direction.

(7) The bioelectrode-equipped apparatus according to (6), wherein an elongation rate at a load of 2.1N per 10mm width measured according to a JIS L 1096 D method is 20% or less for the first extension direction in the electrode placement region of said base fabric.

(8) The bioelectrode-equipped apparatus according to (6), wherein an elongation rate at a load of 2.1N per 10mm width measured according to a JIS L 1096 D method is in the range of 25 to 150% or less for the second extension direction in the electrode placement region of said base fabric.

(9) The bioelectrode-equipped apparatus according to (6), wherein an elongation elastic modulus measured according to a JIS L 1096 E method at a load of 2.1N per 10mm width with one repeat frequency is 80% or more for the second extension direction in the electrode placement region of said base fabric.

(10) The bioelectrode-equipped apparatus according to (6), wherein the angle that the first extension direction and the second extension direction form with each other is in the range of 60-90 degrees.

(11) The bioelectrode-equipped apparatus according to (6), wherein a second insulating layer is provided between the wiring and the base fabric.

(12) The bioelectrode-equipped apparatus according to (6), wherein a skin-side fabric is provided on the surface of the base fabric where the bioelectrode is provided which is the surface to be in contact with a skin in said electrode placement region.

(13) The bioelectrode-equipped apparatus according to (6), wherein said bioelectrode comprises a fabric having a metal film.

(14) The bioelectrode-equipped apparatus according to (6), wherein said bioelectrode is any one of the bioelectrodes according to (1) to (5).

(15) The bioelectrode-equipped apparatus according to (6), wherein said wiring comprises a conductive resin composition.

(16) The bioelectrode-equipped apparatus according to (6), wherein a restraining part is provided along the first extension direction.

(17) The bioelectrode-equipped apparatus according to (6), wherein said apparatus is in a cylindrical shape or has a shape that can be fixed by winding around an arm or a leg.

**Effect of the Invention**

The present invention makes it possible to obtain a biological signal having few artifacts by achieving a state in which more moisture than necessary is not present in a boundary part between the surface of bioelectrode and the skin, and as a result, to obtain a bioelectrode which enables a highly accurate measurement of biological signals.

The present invention also makes it possible to obtain a bioelectrode which has no possibility of a short circuit between adjacent bioelectrodes and has suppressed discomfort at the time of wearing or measurement of biological signals.

In the bioelectrode-equipped apparatus of the present invention comprising a base fabric having an electrode placement region, at least the part of the base fabric composing the electrode placement region comprises an elastic fabric having at least a first extension direction which exhibits relatively low extensibility and a second extension direction which is different in the extension direction from the first extension direction and which exhibits higher extensibility than the first extension direction, and a wiring is formed along the first extension direction exhibiting relatively low extensibility.

Thus, the apparatus of the present invention makes it possible to apply the bioelectrode accurately to a predetermined position of a body regardless of individual differences in the circumference (a thickness) of the applied part, while hardly causing disconnection of wirings even when a force is applied at the time of wearing or the like.

The bioelectrode-equipped apparatus of the present invention can be used effectively as a device to be worn on a body (a supporter) to fix by winding around an arm or a leg so that the bioelectrode being in contact with the skin surface of the wearer, or to wear in a cylindrical shape.

Since the apparatus is used so that the second extension direction which exhibits relatively high extensibility is directed along the circumferential direction of an arm or a leg, the electrode can be in contact with the predetermined position on the skin surface accurately regardless of the individual differences in thickness.

Applying the bioelectrode-equipped apparatus of the present invention and measuring a potential difference between the arbitrary electrodes, impedance, capacitance and the like, allows to know the biological activities and/or the state of a subcutaneous tissue.

In addition, applying an electrical stimulation to a biological body from the electrode allows to perform treatment of paralysis or stiffness and to enhance a training effect of muscles.

The position where the electrode comes in contact with is important for accurate measurement and appropriate electrical stimulation. According to the present invention, the electrode of the apparatus can be in contact with an appropriate position depending on the thickness of the applied position of a body caused by relatively high extensibility of the second extension direction, which enables to perform accurate measurement and appropriate electrical stimulation.

**Brief Description of Drawings**

[Fig.1]

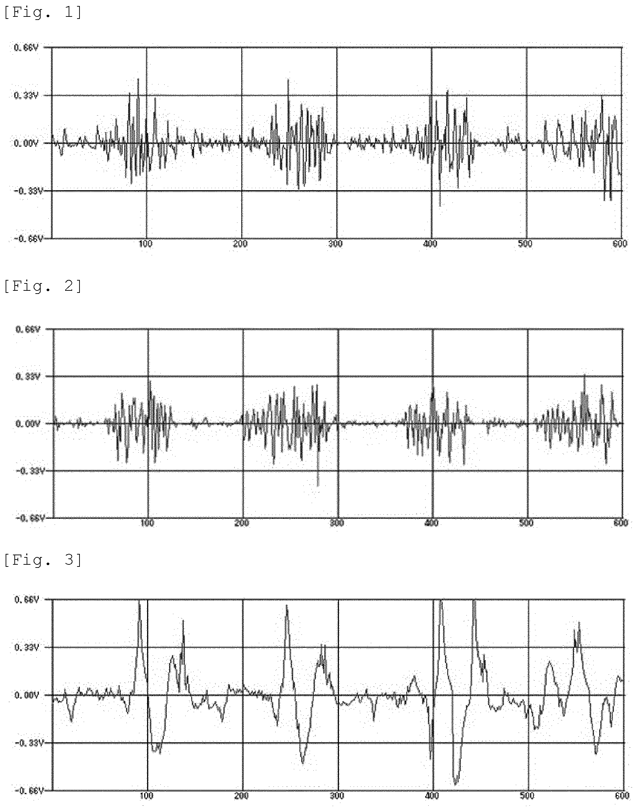

Figure 1 shows EMG (electromyogram) which is obtained by using a bioelectrode of the present invention obtained in Example 1.
[Fig.2]

Figure 2 shows EMG (electromyogram) which is obtained by using a bioelectrode of the present invention obtained in Example 2.

[Fig.3]

Figure 3 shows EMG (electromyogram) which is obtained by using a bioelectrode obtained in Comparative Example 1.

[Fig.4]

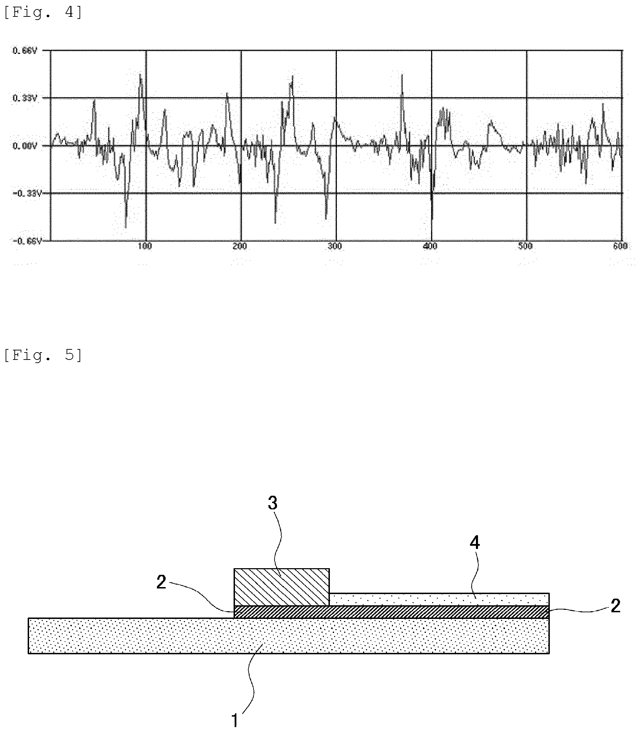

Figure 4 shows EMG (electromyogram) which is obtained by using a bioelectrode obtained in Comparative Example 2.

[Fig.5]

Figure 5 shows a schema of a cross section of the electrode placement region in one embodiment of the bioelectrode-equipped apparatus of the present invention.

[Fig.6]

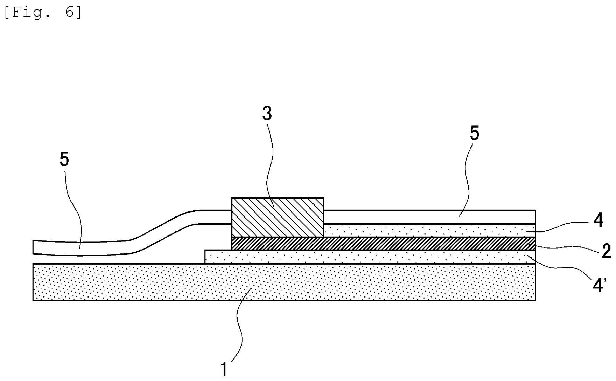

Figure 6 shows a schema of a cross section of the electrode placement region in one embodiment of the bioelectrode-equipped apparatus of the present invention.

[Fig.7]

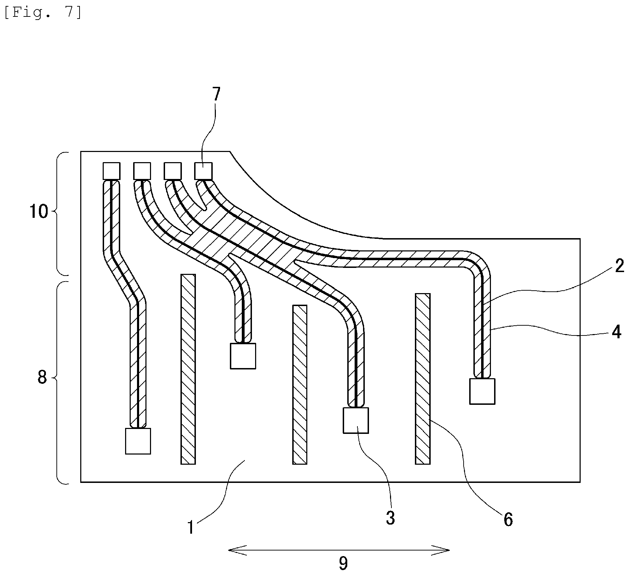

Figure 7 shows a schema of one embodiment of the bioelectrode-equipped apparatus of the present invention.

**Explanation of Reference Letters**

1: Base fabric

2: Wiring

3: Electrode

4: Insulating layer (First insulating layer)

4': Second insulating layer

5: Skin-side fabric

6: Restraining part

7: Terminal

8: Electrode placement region

9: Second extension direction

10: Input/output region

**Modes for Carrying Out the Invention**

I. Bioelectrode

The bioelectrode of the present invention comprises a sheet-like structure containing a conductive fiber and a water-absorbing resin applied to said sheet-like structure.

The bioelectrode of the present invention makes it possible to prevent moisture, which has been uptaken from the skin or has been applied to the bioelectrode prior to measurement of biological signals, from exuding onto the outer surface of the bioelectrode, whereas a certain amount of moisture is contained in the vicinity of the surface of the bioelectrode.

As a result, there is no possibility of causing discomfort or a short circuit between adjacent bioelectrodes because no more moisture than necessary is present between the skin and the bioelectrode.

Since moisture is retained inside the bioelectrode and in the vicinity of the inner surface of the bioelectrode, highly accurate measurement of biological signals can be performed without influence of artifacts.

1. Sheet-like Structure

(1) Conductive Fiber

The sheet-like structure of the present invention contains a conductive fiber as a component. Examples of the conductive fiber include a metal wire, a fiber composed of a conductive polymer and a fiber composed of a non-conductive polymer to which conductivity is imparted.

(i) Metal Wire

Examples of metal for the metal wire include copper, silver, gold, iron, stainless steel, nickel, chromium, tin and aluminum. Preferable examples among them include copper and stainless steel in terms of high versatility.

Although the thickness of the metal wire is not particularly limited, it has preferably a diameter of 10-200 $\mu$m.

(ii) Conductive Polymer

Examples of the conductive polymer include "PEDOT/PSS" which is a conductive thiophene-based polymer that is poly-(3,4-ethylenedioxy thiophene) [PEDOT] doped with poly-(4-styrene sulfonic acid) [PSS], polyacetylene, poly-(p-phenylene vinylene), polypyrrole, polythiophene, polyaniline and poly(p-phenylene).
Preferable examples thereof include PEDOT/PSS and polyaniline. Particularly preferred is PEDOT/PSS. Although the fiber diameter of the conductive polymer is not particularly limited, it has preferably a diameter of 10-200 $\mu$m.

(iii) Fiber composed of a non-conductive polymer to which conductivity is imparted:

A fiber composed of a non-conductive polymer to which conductivity is imparted can also be used as a conductive fiber. The fiber composed of a non-conductive polymer, hereinafter a non-conductive polymer fiber, may be appropriately selected from conventionally known fibers. A synthetic fiber is preferable in terms of strength and versatility.
Examples of the synthetic fibers include a nylon fiber, a vinylon fiber, a polyester fiber, a polyolefin fiber, an acryl fiber, a vinylidene chloride fiber, a polyurethane fiber, and an aramid fiber. Preferable examples among them include a nylon fiber, a polyester fiber, and an aramid fiber. Particularly preferred is a polyester fiber.
Although the fiber thickness of the non-conductive polymer fiber is not particularly limited, it has preferably a diameter of 10-200 $\mu$m or a fineness of 2-400 dtex.
The non-conductive polymer fiber may be a monofilament composed of the above-described fibers or may be a multifilament composed of two or more monofilaments. The number of filaments composing the multifilament is preferably 5-100.
Examples of means for imparting conductivity to the non-conductive polymer fiber include a method of coating the surface of the non-conductive polymer fiber with a conductive polymer, a method of coating a conductive coating material containing conductive particles on the surface of the non-conductive polymer fiber, and a method of forming a metal film on the surface of the non-conductive polymer fiber by metal plating or the like.
Regarding the method of coating the surface of the non-conductive polymer fiber with a conductive polymer, examples of the conductive polymers used for coating include PEDOT/PSS, polyacetylene, poly(p-phenylenevinylene), polypyrrole, polythiophene, polyaniline, and poly(p-phenylene).
Examples of the means for coating the conductive polymer include an extrusion coating and a dipping coating. Although the thickness of the conductive polymer coated film is not particularly limited, it has preferably a thickness of 0.01-200 $\mu$m. The ratio of the conductive polymer to the total amount of the non-conductive polymer fiber to which conductivity is imparted by these means for coating is preferably 0.1-90 mass%, although not particularly limited.
As the conductive coating material to be used for the method of coating a conductive coating material containing conductive particles on the surface of the non-conductive polymer fiber, a coating material wherein conductive particles such as a particle of metal such as copper, silver, nickel and tin, a silver-silver oxide particle and carbon black are dispersed in a synthetic resin such as an acrylic resin or a polyurethane resin can be used.
Although the particle diameter of the conductive particles is not particularly limited, it is preferably 0.01-100 $\mu$m, particularly preferably 0.1-50 $\mu$m. The blending ratio of the conductive particles to the total amount of the synthetic resin is preferably 5-99 mass%, particularly preferably 10-95 mass%.
Examples of the means for coating the conductive coating material include an extrusion coating and a dipping coating. Although the thickness of the coated film of the conductive coating material is not particularly limited, it has preferably a thickness of 0.01-200 $\mu$m. The ratio of the conductive coating material to the total amount of the non-conductive polymer fiber to which conductivity is imparted by these means for coating is preferably 0.1-90 mass%, although not particularly limited.
Regarding the method of forming a metal film on the surface of the non-conductive polymer fiber by metal plating or the like, examples of the metals for metal plating include copper, silver, gold, platinum, nickel, chromium, and tin. Preferable examples thereof include copper, silver, and gold in terms of excellent conductivity. Particularly preferable examples thereof include silver in terms that the bioelectrode has an excellent corrosion resistance against an electrolyte aqueous solution such as sweat and has a low resting potential at the time of contacting with sweat, and that noise is hardly mixed in the biological signals to be measured.
Preferable examples of methods for forming a metal film include metal plating. Means for metal plating may be selected from conventionally known methods such as electrical metal plating and electroless metal plating.
Regarding the method of forming a metal film on the surface of the non-conductive polymer fiber, the ratio of the metal film (the amount of applied metal) to the total amount of the non-conductive polymer fiber to which conductivity is imparted is preferably 5-40 mass%, more preferably 10-30 mass%, although not particularly limited.
Among the above-described means for imparting conductivity to the non-conductive polymer fiber, the method of forming a metal film by metal plating is preferable in terms that enough conductivity required as an electrode can easily be obtained.

(iv) Summary

The conductive fiber contained in the sheet-like structure of the present invention is preferably a synthetic fiber having a metal film on its surface. It enables to obtain a bioelectrode having small surface resistance and high sensitivity.

(2) Sheet-like Structure

(i) Composition of Sheet-like Structure

The materials composing the sheet-like structure of the present invention may be fiber materials such as a nylon fiber, a vinylon fiber, a polyester fiber, a polyolefin fiber, an acrylic fiber, a vinylidene chloride fiber, a polyurethane fiber, and an aramid fiber, in addition to the above-described conductive fibers.

Although fineness of the above-described fiber materials composing the sheet-like structure is not particularly limited, it is preferably 2-100 dtex, more preferably 5-60 dtex.

The fiber may be a monofilament or may be a multifilament composed of two or more monofilaments. The number of filaments composing the multifilament is preferably 2-100. Among them, it is preferable to use a monofilament having elasticity (an elastic yarn), and it is particularly preferable to use a polyurethane elastic yarn.

The structure of the sheet-like structure is not particularly limited. Examples of preferable structures include fiber structures obtained by using the above-described fiber materials such as a woven fabric, a knitted fabric, and a non-woven fabric. A particularly preferable structure is a knitted fabric.

Although the thickness of the sheet-like structure is not particularly limited, it has preferably a thickness of 0.1-4.0 mm, more preferably 0.5-2.0 mm, in terms of shape stability as a bioelectrode and improvement of adhesiveness of the bioelectrode to the skin while increasing mechanical strength.

The sheet-like structure of the present invention contains the above-described conductive fiber at least as a part of components. At least a part of the fibers composing the sheet-like structure is the above-described conductive fiber.

The content of the conductive fiber to the total weight of the sheet-like structure is preferably 30-100 mass%, more preferably 60-100 mass%, most preferably 90-100 mass%.

(ii) Process for Manufacturing the Sheet-like Structure

The process for manufacturing the sheet-like structure of the present invention is not particularly limited. When the conductive fiber is a metal wire, a fiber material for preparing the sheet-like structure wherein a metal wire is included as a part can be molded into a sheet appropriately. Examples of the means for molding into a sheet include weaving, knitting, molding by using a binder resin, and molding by heat fusion.

When the conductive fiber is composed of a conductive polymer, a fiber material for producing the sheet-like structure in which a fiber composed of a conductive polymer is included as a part can be molded into a sheet in the same manner appropriately.

In the case that a non-conductive polymer fiber to which conductivity is imparted is used as the conductive fiber, a fiber material for producing the sheet-like structure in which a non-conductive polymer fiber to which conductivity is imparted is included as a part can be molded into a sheet in the same manner appropriately.

The sheet-like structure containing a conductive fiber can also be obtained by winding a conductive fiber around a fiber material for producing the sheet-like structure as a core yarn (or by covering the core yarn by the conductive fiber) to make a covering yarn containing a conductive fiber, and then using the covering yarn for molding into a sheet by weaving, knitting, molding by using a binder resin, or molding by heat fusion, or the like.

In this case, the fiber material for producing a sheet-like structure is preferably an elastic monofilament (an elastic yarn) , more preferably a polyurethane elastic yarn. The conductive fiber is preferably a metal plated yarn obtained by plating metal such as silver onto a synthetic fiber such as a polyester fiber.

The fineness of the fiber material to be used as a core yarn for the covering yarn is preferably 10-100 dtex, more preferably 10-60 dtex.

The synthetic fiber used for a conductive fiber for the covering yarn is preferably a multifilament having the number of filaments of 5-100, and having a total fineness of 10-100 dtex.

The content of applied metal in the metal plated yarn obtained by plating metal onto the synthetic fiber is preferably 5-40 mass%, more preferably 10-30 mass%.

The mass ratio of the core yarn and the conductive fiber is preferably 5:95 - 25:75. The content of the conductive fiber to the total amount of the covering yarn is preferably 75-95 mass%.

In the case of using a non-conductive polymer fiber, another method of molding a non-conductive polymer fiber into a sheet before imparting conductivity and then imparting conductivity thereto can be employed.

Examples of means for molding a non-conductive polymer fiber into a sheet include weaving, knitting, molding by using a binder resin, or molding by heat fusion.

Examples of means for imparting conductivity to the sheet obtained by molding a non-conductive polymer fiber include the same methods as the above-described means for imparting conductivity to the non-conductive polymer fiber. Among them, it is preferable to use a method of forming a metal film by means of metal plating.

The bioelectrode of the present invention can contain a considerable amount of moisture in the vicinity of the surface thereof. The reason why the bioelectrode is excellent in the function of containing moisture is not necessarily clear. It may be not only the presence of a water-absorbing resin but also a significant effect of a fine structure of fibers composing the sheet-like structure. A moisture retainment caused by so called capillary action may occur depending on the size, volume and/or distribution of voids existing inside of the sheet-like structure.

The sheet-like structure is preferably a knitted fabric. A water-absorbing resin can be applied to the inner void of the sheet-like structure composed of a knitted fabric, which enables to retain moisture in the vicinity of the inner surface of the bioelectrode and to suppress exuding of moisture to the outer surface of the bioelectrode. As a result, a bioelectrode capable of measuring highly accurate biological signals can be obtained.

Although the density of the knitted fabric is not particularly limited, it is preferable that the knitted fabric has a course of 20-70 courses/2.54 cm and a wale of 30-100 wales/2.54 cm. The weight per unit of the knitted fabric is preferably 80-300 g/m$^2$.

The structure of the above-described knitted fabric is not particularly limited. Examples of the structures include jersey stitch, pearl knitting, waffle knitting, moss stitch, fraise stitch, smooth knitting, double tricot knitting, double raschel knitting and the like. Among them, smooth knitting is preferable in terms of having a suitable thickness and dense inner voids with excellent stretchability. Furthermore, triple layer smooth knitting and quadruple layer smooth knitting are most preferable. The thickness of the knitted fabric is preferably 0.1-4.0 mm, more preferably 0.5-2.0 mm.

2. Water-absorbing Resin

According to the present invention, a water-absorbing resin is applied to the sheet-like structure. The water-absorbing resin comprises a material having a high affinity for water. The water-absorbing resin is required to have a certain level of moisture retention capability. In particular, it is desirable to have ability of absorbing and retaining moisture in an amount of 0.5-20 mass times, preferably 0.5-10 mass times, more preferably 1.0-5 mass times of its own weight based on moisture retention capability measured according to a JIS K 7223-1996 method.

Examples of the water-absorbing resin as described above include various kinds of hydrogel materials such as a polymer or a copolymer of acrylic acid and/or acrylate, and a crosslinked product thereof. Specifically, one or more of the following (a)-(g) can be employed.

    (a) Acrylic acid polymer
    (b) Copolymer of acrylic acid and other monomers
    (c) Acrylic acid salt polymer
    (d) Copolymer of acrylic acid salt and other monomers
    (e) Copolymer of acrylic acid and acrylic acid salt
    (f) Copolymer of acrylic acid, acrylic acid salt, and other monomers
    (g) Crosslinked product of any of the above-described (co) polymer

Comonomer components (other monomers) of the above-described copolymer is not particularly limited. Examples of comonomers include methacrylic acid, acrylic acid esters and methacrylic acid esters. Examples of salts include alkali metal salts such as a sodium salt and a potassium salt.

Other water-absorbing resins may be used, examples of which include a graft copolymer wherein acrylic acid and/or acrylate polymer or copolymer (any of the above-described (a)-(f)) is introduced as a side chain in a main chain of an arbitrary polymer, and its crosslinked product.

Although the polymer to be used for a main chain of the graft copolymer is not particularly limited, it is preferable to use starch, carboxymethyl cellulose, polyvinyl alcohol, polyvinyl acetate, or the like.

Examples of the water-absorbing resins also include a crosslinked product of starch or carboxymethyl cellulose, a crosslinked product of a hydrolysis product of a starch-acrylic acid salt graft copolymer, a crosslinked product of a vinyl alcohol-acrylic acid salt copolymer, a crosslinked product of a maleic anhydride-grafted polyvinyl alcohol, a crosslinked isobutylene-maleic anhydride copolymer, a saponified vinyl acetate-acrylic acid ester copolymer, and a polyester resin emulsion.

Among them, it is preferable to use a crosslinked product of an acrylic acid and/or acrylic acid salt polymer or copolymer which is corresponding to (g) among the above-described (a) - (g), and a crosslinked product of a graft copolymer wherein acrylic acid and/or acrylic acid salt polymer or copolymer is introduced as a side chain.

Examples of the polymers to be used for a main chain of the graft copolymer include starch, carboxymethyl cellulose, polyvinyl alcohol, and polyvinyl acetate. Preferable salts include a sodium salt.

It is more preferable to use a crosslinked product of an acrylic acid and/or acrylic acid salt polymer or copolymer.

It is further preferable to use a crosslinked product of any one of the acrylic acid polymer (the above-described (a)), the acrylic acid salt polymer (c), and the copolymer of acrylic acid and acrylic acid salt (e). In other words, a crosslinked product of the (co)polymers selected from the group consisting of (a), (c) and (e), hereinafter "a crosslinked product of a polyacrylic acid and/or a salt thereof", is preferably used. Most preferably, a crosslinked product of the copolymer of acrylic acid and acrylic acid salt (a crosslinked product of (e)) is used. Examples of the salts thereof include a sodium salt thereof.

One embodiment of the means for manufacturing the crosslinked product of a polyacrylic acid and/or a salt thereof is a method of neutralizing at least a part of carboxyl groups of the polyacrylic acid to transform into carboxylic acid salt, and then crosslinking at least a part of the remained carboxyl groups by applying a crosslinking agent.

A reactive compound having two or more epoxy groups can be used as a suitable crosslinking agent. Examples of the reactive compounds include polyglycidyl ethers such as glycerol polyglycidyl ether, polyglycerol polyglycidyl ether, sorbitol polyglycidyl ether, trimethylolpropane polyglycidyl ether, and pentaerythritol polyglycidyl ether. Among them, glycerol polyglycidyl ether is preferred.

The usage amount of the crosslinking agent is not particularly limited. When the crosslinking agent is a polyglycidyl ether compound, it is preferable to use the crosslinking agent so that the ratio of "the number of moles of epoxy groups in the polyglycidyl ether compound" : "the number of moles of carboxyl groups in the water-absorbing resin" is in the range of 25:100 - 75:100. "The number of moles of carboxyl groups" here means a total number of moles of carboxyl groups including carboxy groups neutralized to form a salt.

When the usage amount of the crosslinking agent is too small, the water-absorbing resin might easily remove out of the bioelectrode due to weak adhesion to the sheet-like structure at the time of water absorption. When the usage amount is too large, a texture of the bioelectrode might become hard.

Moisture retention capacity of the crosslinked product of a polyacrylic acid and/or a salt thereof is depending on the number of carboxyl groups which are neutralized to form carboxylates. Therefore, the carboxylate equivalent in the crosslinked product is preferably 150-1,500 g/eq, more preferably 300-1, 000 g/eq. By selecting a crosslinked product of a polyacrylic acid and/or a salt thereof whose carboxylate equivalent is in the above-described range as a water-absorbing resin, the moisture retention index of the bioelectrode can be adjusted to 0.8 or more. The carboxylate equivalent can be adjusted to the above-identified range by appropriately selecting the usage amount of a neutralizer (such as sodium hydroxide in the case of a sodium salt) and a crosslinking agent.

A crosslinked product of polyacrylic acid salt graft copolymer can also be obtained by neutralize at least a part of carboxyl groups of the polyacrylic acid salt graft copolymer to transform into carboxylic acid salt, and then applying a crosslinking agent.

A polyester resin emulsion can also be suitably used. For example, a polyester resin composition containing an anionic surfactant which is sometimes referred to as an "anionic special polyester resin" can be used.

The above-described examples of water-absorbing resins can be used singly or in a combination of two or more kinds thereof.

The average molecular weight of the water-absorbing resin is not particularly limited. Generally, it is preferable to use a water-absorbing resin having an average molecular weight of 4,000-100,000.

Applying the water-absorbing resin to the sheet-like structure makes it possible to control the moisture retention index of the bioelectrode easily and to manufacture a bioelectrode of the present invention by a simple process.

Examples of means for applying the water-absorbing resin to the sheet-like structure include a method of dipping the sheet-like structure into a treatment liquid in which the water-absorbing resin is dissolved or dispersed in a solvent and a method of spraying said treatment liquid to the sheet-like structure by a sprayer or the like, but not particularly limited.

After applying, the solvent contained in the treatment liquid is removed by vaporizing or the like to form a thin film of the water-absorbing resin coating the sheet-like structure.

The fibers composing the sheet-like structure after drying are not necessarily coated with the water-absorbing resin completely. The water-absorbing resin may also be only adhered on the fibers discontinuously.

The solvents used for dissolving or dispersing the water-absorbing resin in the treatment liquid is not particularly limited if it is possible to dissolve or disperse a water-absorbing resin. Examples of the solvents include water and organic solvents. Examples of organic solvents include alcohols such as methanol, ethanol and 2-propanol, ketones such as acetone and methylethylketone, N,N-dimethylformamide, dimethyl sulfoxide, N-methyl pyrrolidone, and γ-butyrolactone. The concentration of the water-absorbing resin in the treatment liquid is preferably 1-40 mass%, but not particularly limited.

An application amount of the water-absorbing resin to the total amount of the sheet-like structure after applying the water-absorbing resin is preferably 0.5-100 g/m$^2$, more preferably 10-80 g/m$^2$. The film thickness of the thin film of the water-absorbing resin coated on the sheet-like structure is preferably 0.1-40 $\mu$m, more preferably 0.1-20 $\mu$m, but not particularly limited.

The bioelectrode of the present invention wherein the water-absorbing resin is applied to the sheet-like structure is characterized in that a certain amount of moisture can be contained in the vicinity of the surface thereof. In particular, it is essential that the bioelectrode of the present invention has a moisture retention index of 0.8 or more, preferably 0.9 or more.

The moisture retention index according to the present invention is described as follows:

0.01 g of pure water is gently placed on the surface of a sample of the bioelectrode which is placed on an approximately horizontal table, and the sample is allowed to stand for 30 seconds. Then, a quantitative filter paper No.5A, manufactured by TOYO ROSHI KAISHA, LTD., is placed overlapping on the position where pure water is placed. Then, a silicone rubber sheet having a diameter of 50 mm and a thickness of 0.5 mm and an iron plate having a diameter of 50 mm (total mass of 100g) are placed overlapping on the quantitative filter paper so that the pressure becomes 0.5 kPa, and the sample is further allowed to stand for 60 seconds. The ratio of a pure water retained in the sample of the bioelectrode is calculated by the following formula 1 based on the mass of pure water absorbed in the quantitative filter paper.

$$\mathrm{MRI} = 1 - \mathrm{PW(g)}/0.01 \qquad \cdots\cdots (\mathrm{Formula}\ 1)$$

MRI: Moisture Retention Index
PW: Mass of pure water absorbed in the quantitative filter paper (g)

[Formula 1]

$$\text{Moisture Retention Index} = 1 - \frac{\text{Mass of pure water absorbed in the quantitative filter paper (g)}}{0.01}$$

Although it is not necessarily clear why the moisture retention function in the vicinity of the bioelectrode surface of the present invention is as high as to show the moisture retention index as defined above of 0.8 or more, various conditions such as a fine structure of the sheet-like structure and an adhesion state of the water-absorbing resin are considered to affect the function.

Regarding the fine structure of the sheet-like structure, it is considered that moisture retention caused by capillary action occurs according to the size, volume, distribution, or the like of voids present inside of the sheet-like structure. Furthermore, the inner surface area of the sheet-like structure may also have a significant influence. Therefore, a desired moisture retention index can be obtained by selecting a proper condition concerning a fiber structure of the sheet-like structure (preferably a knitted fabric), a thickness of fibers such as a fineness, the number of filaments, voids between fibers (a density of a knitted fabric), a basis weight of a knitted fiber or the like.

A desired moisture retention index can also be obtained by selecting a proper condition concerning a type of the water-absorbing resin, moisture retention capacity of the water-absorbing resin itself, an application amount or a thickness of the water-absorbing resin applied to the sheet-like structure, an adhesion state of the water-absorbing resin to the sheet-like structure or the like. The adhesion state of the water-absorbing resin may be a form of the water-absorbing resin adhering to the sheet-like structure such as a water globule form or the like.

According to the present invention, it is required to keep the moisture retention index of the bioelectrode in the range of 0.8 or more by appropriately combining the above-described conditions.

Keeping the moisture retention index in the range of 0.8 or more enables to prevent moisture from exuding onto the outer surface of the bioelectrode, while retaining moisture inside or in the vicinity of the inner surface of the bioelectrode.

As a result, there is no possibility of causing discomfort because no more moisture than necessary is present between the skin and the bioelectrode, and there is no possibility of a short circuit even when a plurality of bioelectrodes are close to each other.

Since moisture is retained inside and in the vicinity of the inner surface of the bioelectrode, highly accurate biological signals can be measured without influence of artifacts.

Since the bioelectrode of the present invention comprises a sheet-like structure containing a conductive fiber, it is freely cuttable in a conventional manner for cutting a fiber fabric. Therefore, it can be cut into a desired size and shape to apply to the inner side of a body wearing device that is the side that comes in contact with the skin of a body. Since the bioelectrode comprises a sheet-like structure containing a conductive fiber, a metal thin wire for input/output of signals can also be connected to the bioelectrode by sewing.

II. Bioelectrode-equipped Apparatus

The bioelectrode-equipped apparatus (a supporter) of the present invention comprises a fabric structure having an electrode placement region provided in at least a part thereof, which includes a base fabric having an elastic property, a wiring formed on the surface of said base fabric, a bioelectrode provided at the terminal end of said wiring, and an insulating layer for covering said wiring. The surface of the base fabric here means a surface on which a wiring and a bioelectrode are provided. The back side of the base fabric means the opposite side of said surface thereof.

1. Electrode Placement Region

The electrode placement region according to the present invention is a region including at least a wiring, a bioelectrode and an insulating layer provided on the base fabric, wherein the wiring is formed along the first extension direction of the base fabric.
Each of the figures 5 and 6 shows a schema of a cross section of the electrode placement region in one embodiment of the bioelectrode-equipped apparatus of the present invention. As shown in the figure 5, the electrode placement region at least comprises a wiring 2, an electrode 3, and an insulating layer 4 provided on the base fabric 1, wherein the wiring 2 is formed along the first extension direction of the base fabric 1. The electrode 3 is provided in the vicinity of the terminal end of the wiring 2 and is not covered with the insulating layer 4.

(1) Base Fabric

The base fabric according to the present invention is composed of a fabric having an elastic property in terms of wearability to a living body and adhesion of the bioelectrode to a skin. Any fabrics exhibiting extensibility in at least one direction can be used. It may be a fabric exhibiting extensibility in two or more directions, or may be a fabric exhibiting extensibility in every direction.
In terms of followability to a skin, it is preferable to use a fabric having excellent extensibility in every direction except for the wiring direction. For example, a base fabric exhibiting extensibility in every direction can be used by limiting an elongation only in the wiring direction by means of sewing or the like.
A material composing the fabric having an elastic property is not particularly limited if it is a fabric used typically for clothing. Examples of the fabrics include a woven fabric, a non-woven fabric, and a knitted fabric. It is also preferable to select a material excellent in flexibility as well in terms of adhesion to the bioelectrode and wearability.
Examples of the fabrics include, in particular, a fabric made of synthetic fibers such as a polyester fiber, a polyamide fiber, a polyurethane fiber and a polyethylene fiber, a fabric made of natural fibers such as cotton, hemp and silk, a fabric made of blended fibers and mixed fibers thereof. The fabric can be composed of a single kind or two or more kinds of these fibers.
The base fabric can be composed of a monolayer or a multilayer. In the case of a multilayer, the materials forming each layer may be the same with or different from each other.
The thickness of the base fabric is not particularly limited. From the viewpoint of shape stability of the bioelectrode and the improvement of adhesion of the bioelectrode to a skin while enhancing mechanical strength, the thickness of the base fabric is preferably 0.05-1.00 mm, more preferably 0.1-0.5 mm.
The base fabric at least has, in its electrode placement region, a first extension direction which exhibits relatively low extensibility and a second extension direction different from the first extension direction which exhibits higher extensibility than the first extension direction.
The first extension direction and the second extension direction which have different directions from each other may be described in other words that the two directions are not parallel to each other, or that they are crossing with each other.
The first extension direction is preferably a direction having the lowest elongation rate in the base fabric.
As an indication of extensibility of the first extension direction, an elongation rate at a load of 2.1N per 10 mm width measured according to a JIS L 1096 D method may be 20% or less, preferably 15% or less, most preferably 10% or less. Although the lower limit of the elongation rate is not particularly limited, the elongation rate of the first extension direction is preferably near 0%.
As an indication of extensibility of the second extension direction, an elongation rate measured in the same manner according to the above-described JIS method is preferably 25-150%, more preferably 30-100%, most preferably 30-70%.

An elongation elastic modulus for the second extension direction measured according to a JIS L 1096 E method at a load of 2.1N per 10 mm width with one repeat frequency is preferably 80% or more, more preferably 85% or more. When an elongation elastic modulus for the second extension direction is 80% or more, a proper tightening effect can be obtained at the time of applying the bioelectrode-equipped apparatus of the present invention, preventing the electrode positions from being displaced during use. The elongation elastic modulus for the first extension direction is not particularly limited.

The first extension direction and the second extension direction are not parallel to each other, but are indicating different directions. It can be said that they are crossing with each other.

An angle formed by the two directions on an acute angle side, as an indication, is preferably 60-90 degrees, more preferably approximately 90°which is the state that the two directions are crossing in a direction substantially orthogonal to each other, but not particularly limited.

In the case that the base fabric is a common woven fabric consisting of a warp and a weft, the warp and the weft are mutually crossing (orthogonal to each other), and the warp and weft directions may form a first extension direction and a second extension direction of the present invention. Therefore, when a woven fabric is used as a base fabric, by changing the extensibility of the warp and the weft, a first extension direction and a second extension direction having desired extensibility can be formed.

For example, when a woven fabric is formed by using a warp having almost no extensibility and a weft containing a fiber exhibiting extensibility such as a polyurethane elastic yarn, the longitudinal direction of warp is a first extension direction and the longitudinal direction of weft is a second extension direction. The wiring can be formed along the longitudinal direction of warp which is the first extension direction.

When using, as a base fabric, a fabric exhibiting high extensibility in two or more directions or in every direction such as a knitted fabric which has significant extensibility in two directions crossing each other such as vertical and horizontal directions, it is preferable to provide a restraining part using a restraining means for suppressing elongation in one direction in the electrode placement region. The restraining part makes it possible to provide at least one direction as a first extension direction exhibiting low extensibility and a wiring can be formed along said direction.

Examples of restraining means for suppressing elongation include a method of sewing along the direction desired to suppress elongation by using a yarn having low extensibility and a method of adhering a fabric, a film or a sheet having low elongation along the direction desired to suppress elongation.

Examples of yarns for sewing having low extensibility include a polyester fiber, a nylon fiber, and an aramid fiber.

Examples of fabrics having low elongation include a polyester woven fabric, a nylon woven fabric, and an aramid woven fabric. Examples of materials for a film or a sheet having low elongation include a polyurethane resin, a polyester resin, a polyethylene resin, and a polyimide resin.

In order to prepare an elastic fabric having a first extension direction and a second extension direction with different extensibility from each other by weaving, a method of weaving by using a covering yarn in which a polyester yarn or a nylon yarn is wound around a core yarn composed of a polyurethane elastic yarn as a yarn for the second extension direction having relatively high extensibility and a polyester yarn or a nylon yarn as a yarn for the first extension direction having relatively low extensibility can be employed to form a fabric.

When the apparatus of the present invention is a device such as a supporter usable in fixing a body by winding around an arm or leg so that a bioelectrode is in contact with the surface of a skin of the arm or leg, or used by wearing in a cylindrical shape, it is desirable to use the apparatus so that the second extension direction of the base fabric is directed along the circumferential direction of the arm or leg. Therefore, the apparatus may be worn so that the first extension direction crossing with the second extension direction is directed along the direction crossing with the circumferential direction of the arm or leg that is the longitudinal direction of the arm or leg.

(2) Wiring

The wiring according to the present invention is formed on the surface of the base fabric. A bioelectrode is provided at one terminal end of the wiring and an external connecting terminal is connected electrically to the other terminal end thereof, so that the bioelectrode can be connected to the external connecting terminal (an input/output terminal of signals) . The external connecting terminal connects electrically to the external device to perform input/output of signals.

In the electrode placement region, the wiring should be formed along the first extension direction on the base fabric. Forming the wiring along the second extension direction might cause disconnection of the wiring as the base fabric extends.

The wiring can be formed by pasting a cut film of a metal plated fabric, a metallic foil, or a coated film of a conductive resin composition. It can also be formed by printing on the fabric directly using a conductive ink comprising a conductive resin composition or the like.

It is preferable to form a wiring by printing a conductive ink in terms of preventing disconnection.

The metal plated fabric can be obtained by depositing metal such as copper, silver, nickel, tin or the like on a polyester woven fabric by an electroplating method or an electroless plating method each singly or in combination sequentially.

Examples of metals for the metallic foil include copper, silver, nickel and tin. The thickness of the metallic foil is preferably 1-30 μm, although not particularly limited.

Examples of conductive resin compositions for a conductive ink include a known commercial product and a composition obtained by dispersing conductive particles in a solvent solution wherein a synthetic resin such as an acrylic resin or a polyurethane resin is dissolved in a solvent.

Examples of conductive particles include a silver particle, a nickel particle, and a carbon particle.

Examples of solvents include water; alcohols such as ethanol and isopropyl alcohol; ketones such as methylethylketone, methyl isobutyl ketone and cyclohexanone; aromatic hydrocarbons such as toluene and xylene; glycol derivatives such as propyleneglycol monomethylether acetate, diethyleneglycol monoethylether acetate, and diethyleneglycol monobuthylether acetate; γ-butyrolactone, N-methyl methylpyrrolidone, N,N-dimethylformamide, dimethyl sulfoxide, dimethyl acetamide, dimethyl carbonate, dioxane, tetrahydrofuran, and solvent naphtha.

Examples of preferable solvents among them include ketones such as methylethylketone, methyl isobutyl ketone and cyclohexanone; aromatic hydrocarbons such as toluene and xylene; glycol derivatives such as propyleneglycol monomethylether acetate, diethyleneglycol monoethylether acetate, and diethyleneglycol monobuthylether acetate; and γ-butyrolactone.

The content of a solvent to the total amount of the conductive resin composition is 5-95 mass% in general.

Examples of synthetic resins used for the conductive resin composition include a polyester resin in addition to the acrylic resin and the polyurethane resin as described above.

The blending ratio of the conductive particles to the solid component in the conductive resin composition is preferably 50-95 mass%, although not particularly limited.

Examples of the conductive inks other than the conductive resin composition include a conductive polymer such as PEDOT/PSS which is poly-(3,4-ethylenedioxy thiophene) [PEDOT] doped with poly-(4-styrene sulfonic acid) [PSS]. Examples of means for printing include an inkjet printing method and a screen-printing method.

The thickness (diameter) and length of the wiring can be determined depending on the condition of a body part where the bioelectrode-equipped apparatus is applied, the type of electrical signals, a size, condition, and type of the bioelectrode-equipped apparatus and the like, but not particularly limited. Preferably, the thickness of the wiring is 500-5,000 μm, more preferably 1,000-3,000 μm.

(3) Bioelectrode

For measuring biological signals and applying an electrical stimulation, a bioelectrode that comes into contact with a body part where the apparatus is applied such as a skin is used. The bioelectrode is connected to a wiring and is further connected electrically to a terminal via the wiring, which enables the external output of biological signals and the application of an electrical stimulation to a living body. One bioelectrode is provided to one wiring.

Various bioelectrodes can be used according to the condition of a body part where the apparatus is applied and the type of bioelectrical signals. Examples of the bioelectrodes to be used in the present invention include, in particular, a base material provided with a metal film and a base material coated with a conductive coating material.

Most preferable examples of bioelectrodes include a bioelectrode of the present invention comprising a sheet-like structure containing a conductive fiber and a water-absorbing resin applied to said sheet-like structure, which has a moisture retention index of 0.8 or more, as described above.

Preferable examples of a sheet-like structure containing a conductive fiber include a fiber structure obtained by molding a fiber material containing a synthetic fiber having a metal film on the surface thereof. Examples of water-absorbing resins include a crosslinked product of a polyacrylic acid and/or a salt thereof.

In most cases of a bioelectrode-equipped device, a wiring is formed on the opposite side of the surface being in contact with a body part where the apparatus is applied such as a skin, and therefore, it is usually desired that the bioelectrode has conductivity also in the thickness direction. Considering this point, it is preferable to use a porous material as the base material composing the bioelectrode, a porous base material wherein a metal film is formed to the interior thereof.

In terms of wearability (texture) and adhesiveness, in addition, it is preferable to use a material having excellent cushioning (elasticity).

Examples of the porous base materials include a fabric such as a woven fabric, a knitted fabric and a non-woven fabric and a foam made of a polymeric material.

Examples of metals consisting a metal film include copper, silver, nickel and tin. Examples of means for forming a metal film in a porous base material extending into the inside thereof include an electrometal plating and an electroless metal plating.

Examples of conductive coating materials include a coating material wherein conductive particles such as a particle of metal such as copper, silver, nickel and tin, a silver-silver oxide particle and carbon black are dispersed in a synthetic resin such as an acrylic resin or a polyurethane resin. A coating material comprising a conductive polymer such as PEDOT/PSS can also be used.

Examples of means for forming a bioelectrode on the base fabric include a method of pasting a bioelectrode on a predetermined position by using a conductive adhesive or the like or by applying a hotmelt adhesive followed by heating, pressuring, and then cooling.

As a conductive adhesive, known commercial products such as a conductive paste named "DOTITE D-500", manufactured by FUJIKURA KASEI CO., LTD., can be used.

As a hotmelt adhesive, known commercial products such as a polyurethane hotmelt sheet named "Ecellent SHM101", manufactured by Sheedom Co., Ltd., can be used.

The shape (size) and thickness of the bioelectrode can be determined according to the condition of a body part where the bioelectrode-equipped apparatus is applied, the type of electrical signals, the condition and type of the bioelectrode-equipped apparatus and the like, although not particularly limited.

Preferably, the shape of the bioelectrode may be a round shape, a square shape or the like, and the size thereof is 5-100mm $\times$ 5-100mm, the thickness thereof is 0.5-5.0 mm.

(4) Insulating Layer

According to the present invention, the insulating layer is provided on the side of a body part where the apparatus is applied (the skin side) of a fabric structure. That is, the insulating layer is provided on the side of the wiring opposite to the base fabric so as to cover the wiring, which enables to prevent the wiring from coming into contact with the skin.

The insulating layer is not provided at the electrode part. Therefore, the electrode part is exposed on the surface of the fabric structure which is the side to be in contact with a body part where the apparatus is applied such as a skin, which enables the bioelectrode to be in contact with the body part where the apparatus is applied.

According to the present invention, one bioelectrode is provided to one wiring typically. Therefore, in the area where the bioelectrodes are concentrated, the wirings are also crowded. However, by covering the wiring with an insulating layer, short circuit can be prevented.

The insulating layer can cover only the wiring, and can also cover the whole surface of the base fabric except for the electrode part. From the viewpoint of not impairing the extensibility of the second extension direction, it is preferable to cover only the wiring.

Examples of materials composing the insulating layer include a polyurethane resin, an acrylic resin, a polyester resin and a polyamide resin.

Although the thickness of the insulating layer is not particularly limited, the preferable film thickness thereof is 10-200 $\mu$m, more preferably 20-150 $\mu$m.

It is necessary that the insulating layer is provided on the wiring which is provided on the base fabric, at the side of the wiring opposite to the surface to be in contact with the base fabric, so as to cover the wiring.

In addition, another insulating layer (a second insulating layer) can be provided between the wiring and the base fabric, if necessary.

The insulating layer provided on the wiring at the side of the wiring opposite to the surface to be in contact with the base fabric can be defined as "the first insulating layer".

By providing both the first insulating layer and the second insulating layer, the wiring can be covered with the insulating layer in a manner so as to sandwich the wiring from both the top and bottom sides, or the wiring can be covered to surround its periphery with the insulating layer, which allows accurate measurement and appropriate electrical stimulation without a short circuit even when wetted with sweat or the like.

The fabric structure can be formed by using a wiring covered with the insulating layer to surround its periphery in advance.

A third insulating layer can be provided on the base fabric at the side opposite to the surface where a wiring is formed, which is the back side of the base fabric.

In this case, the wiring can also be covered with the first insulating layer and the third insulating layer in a manner so as to sandwich the wiring from both sides, which allows more accurate measurement and appropriate electrical stimulation while preventing a short circuit caused by sweat or the like.

The materials, the thickness or the like of the second and third insulating layers are same as those of the first insulating layer.

(5) Options

The electrode placement region comprises the above-described wiring, bioelectrode and insulating layer provided on the base fabric. In addition, another fabric for improving texture (a "skin-side fabric") can be provided on the side of bioelectrode which is the surface to be in contact with a body part where the apparatus is applied such as a skin, if necessary.

Providing a skin-side fabric can improve texture at the time of wearing.

The skin-side fabric can be provided on the entire area of the electrode placement region except for the bioelectrode, to cover the wiring and the insulating layer.

In order to fix the skin-side fabric on the base fabric to prevent displacement at the time of wearing, it is desirable to adhere the skin-side fabric partially not on the entire surface or to fix by sewing. When the skin-side fabric is adhered on the entire surface of the base fabric, stiffness may be generated to the bioelectrode-equipped apparatus to cause a possibility of inhibiting extensibility of the second extension direction.

Fig. 6 shows a schema of a cross section of the electrode placement region in one embodiment of the bioelectrode-equipped apparatus in which a second insulating layer and a skin-side fabric are provided. As shown in Fig.6, the second insulating layer 4' is provided on the base fabric 1 and a wiring 2 is provided on the second insulating layer. That is, the second insulating layer 4' is provided between the base fabric 1 and the wiring 2. On the wiring 2, a first insulating layer 4 is further provided and the wiring 2 is covered so as to be sandwiched with the first insulating layer 4 and the second insulating layer 4' . The bioelectrode 3 is provided in the vicinity of the terminal end of the wiring 2 and is not covered with the first insulating layer 4. A skin-side fabric 5 is provided on the first insulating layer 4. The skin-side fabric 5 can also cover the area other than the electrode placement region on the base fabric. The bioelectrode 3 is not covered with the skin-side fabric 5.

Materials composing the skin-side fabric are not particularly limited as far as it is a fabric commonly used for improving texture. Preferable materials include nylon tricot and the like. In order not to inhibit the extensibility of the second extension direction of the base fabric, it is desirable that the skin-side fabric has extensibility equal to or higher than that of the second extension direction of the base fabric.

The thickness of the skin-side fabric can be determined according to the condition of a body part where the bioelectrode-equipped apparatus is applied, the type of electrical signals, a size, condition, and type of the bioelectrode-equipped apparatus and the like, but not particularly limited. Preferably, the thickness of the skin-side fabric is 0.1-1.0 mm.

2. Input/Output Region

According to the present invention, the bioelectrode placed in the above-described electrode placement region is connected electrically to a terminal via a wiring, which enables to perform external output of biological signals and application of electrical stimulation to a living body. The region where input/output of signals is performed by placing terminals is defined as, hereinafter, "input/output region".

Figure 7 shows a schema of one embodiment of the bioelectrode-equipped apparatus of the present invention containing an electrode placement region and an input/output region. In Fig.7, "9" indicates the second extension direction of the base fabric, "8" is the electrode placement region, and "10" is the input/output region. The wiring 2 is provided on the base fabric 1, the bioelectrode 3 is connected to the vicinity of one terminal end of the wiring 2, and the other terminal end thereof is connected to the terminal 7 located in the input/output region 10. The insulating layer 4 is provided along the wiring 2 so as to surround the wiring 2. A restraining part 6 is provided on the base fabric 1 so as to be crossing in a direction substantially orthogonal to the second extension direction 9 (that is the first extension direction), and the wiring 2 is formed extending along the first extension direction whose extensibility is suppressed by the restraining part 6.

In the input/output region, the terminal is connected electrically to an external device to perform input/output of signals. In order to make it easy to connect with the external device, it is preferable to place the terminals in a concentrated manner.

The input/output region can be distinguished with the electrode placement region in that there is no electrode in the input/output region.

Since the terminals is placed in a concentrated manner in the input/output region, the wiring can be placed focusing on a positional relationship with the terminals without considering extensibility of the base fabric.

When the bioelectrode-equipped apparatus is used by winding on an arm or a leg, the input/output region may not be in a cylindrical shape. It may not be used by contacting with the skin either. The input/output region may be protruded in a tongue-like shape from one end of the electrode placement region formed in a cylindrical shape.

For example, the left and right sides of the bioelectrode-equipped apparatus shown in Fig.7 are stuck together to form a cylindrical shape, whereas the input/output region 10 is formed not in a cylindrical shape but in a tongue-like shape protruding from a part of the upper end of the electrode placement region 8 in a cylindrical shape.

It does not matter whether or not the fabric composing the input/output region has extensibility in any direction.

The base fabric used for the electrode placement region and the base fabric used for the input/output region may be the same, and in this case, the electrode placement region and the input/output region can be formed integrally in one common fabric as a base fabric. The preferable thickness, type, elongation rate or the like of the base fabric is the same as those of the above-described base fabric used for the electrode placement region.

The base fabric used for the input/output region may also be different from the base fabric used for the electrode placement region. From the viewpoint of preventing disconnection of a wiring in the input/output region, it is preferable that the fabric consisting of the input/output region does not exhibit too high extensibility. For the purpose of suppressing extensibility of the fabric itself, the entire area of the input/output region can be covered with a film or the like which does not exhibit extensibility. The restraining means used for suppressing extensibility of the electrode placement region as described above can also be used for suppressing extensibility of the input/output region.

III. Summary

1. Bioelectrode

The bioelectrode of the present invention having a moisture retention index of 0.8 or more makes it possible to prevent moisture from exuding onto the outer surface of the bioelectrode, whereas a certain amount of moisture is contained in the vicinity of the surface of the bioelectrode.

As a result, there is no possibility of causing discomfort or a short circuit between a plurality of bioelectrodes provided adjacent to each other, because no more moisture than necessary is present between the skin and the bioelectrode.

Since moisture is retained inside the bioelectrode and in the vicinity of the inner surface of the bioelectrode, highly accurate measurement of biological signals can be performed without influence of artifacts.

Since the bioelectrode of the present invention comprises a sheet-like structure containing a conductive fiber, it is freely cuttable in a conventional manner for cutting a fiber fabric. Therefore, it can be cut into a desired size and shape to apply to the inner side of a body wearing device that is a side to be in contact with the skin of a wearer.

Since the bioelectrode comprises a sheet-like structure containing a conductive fiber, a metal thin wire for input/output of signals can also be connected to the bioelectrode by sewing.

2. Bioelectrode-equipped apparatus

The bioelectrode-equipped apparatus of the present invention can be used as a device to be worn on a living body (a supporter) to fix by winding around an arm or a leg so that the bioelectrode comes into contact with the skin surface of the body, or to wear in a cylindrical shape.

Since the apparatus is used so that the second extension direction which exhibits relatively high extensibility is directed along the circumferential direction of an arm or a leg, the bioelectrode can be in contact with the predetermined position on the skin surface accurately regardless of the individual differences in thickness.

Applying the bioelectrode-equipped apparatus enables accurate measurement of a potential difference between the arbitrary electrodes, impedance, capacitance and the like, which allows to know the biological activities and/or the state of a subcutaneous tissue better.

In addition, electrical stimulation can be applied to an accurate position of a living body, which makes it possible to perform treatment of paralysis or stiffness and to enhance a training effect of muscles efficiently.

**Examples**

The present invention will be described in more detail below referring to examples. Note that the scope of the present invention is not limited by the following examples.

<Example 1>

(1) Preparation of Sheet-like Structure containing Conductive Fiber

A polyethylene terephthalate (PET) yarn having 33 dtex/6 filaments was subjected to silver plating to prepare a silver-plated yarn having a silver deposition amount of 20 mass%.

A single yarn of a polyurethane (PU) elastic yarn named "ROICA"(a registered trademark) having 22 dtex/monofilament, manufactured by Asahi Kasei Corporation, was used as a core yarn.

The core yarn was covered with a single yarn of the silver-plated yarn, twisted in the Z-twist at 650 T/M (the number of twisting; times/m), to obtain a covering yarn composed of a PU yarn and a silver-plated yarn.

A covering machine named "B-3 type", manufactured by Kataokakikaikougyou, was used for covering. The mass ratio of the PU yarn to the silver-plated yarn was 17:83, and the content of the silver-plated yarn to the total amount of the covering yarn was 83 mass%.

A knitted fabric of sheet-like structure containing a conductive fiber was prepared by knitting the covering yarn by a quadruple layer smooth knitting structure with 22 gauges. The knitted fabric thus obtained had a density of 35 courses/2.54cm, 54 wales/2.54cm, and a basis weight of 192 g/m$^2$. The knitted fabric had a thickness of 1.2mm and the size of diameter 35 cm $\times$ length 50 cm in a cylindrical shape.

(2) Preparation of Water-absorbing Resin

1.112 parts by mass of a sodium hydroxide aqueous solution having a concentration of 20 mass% was added to 8 parts by mass of a polyacrylic acid aqueous solution having a concentration of 20 mass% named "Polyacrylic acid 25, 000", manufactured by Wako Pure Chemical Industries, Ltd., which contains polyacrylic acid having an average molecular weight of 25, 000, and they were mixed to convert 25% of carboxyl groups in the polyacrylic acid into sodium salts thereof.

Then, 1.56 parts by mass of glycerol polyglycidyl ether named "DENACOL EX-313", manufactured by Nagase ChemteX Corporation, as a crosslinking agent, and 20.928 parts by mass of water were added thereto and mixed to prepare a water-absorbing resin treatment liquid. The added amount of the crosslinking agent was determined so that the ratio (the number of moles of epoxy groups in the polyglycidyl ether compound) : (the number of moles of carboxyl groups in the water-absorbing resin) becomes 50:100.

The water-absorbing resin thus obtained had ability of absorbing and retaining moisture in an amount of 1.2 mass times its own weight. The equivalent weight of the carboxylic acid sodium salt was 590 g/eq.

(3) Preparation of Bioelectrode

The above-prepared sheet-like structure (a knitted fabric) was immersed in the water-absorbing resin treatment liquid for 0.5 minutes, and then the sheet-like structure (a knitted fabric) containing the water-absorbing resin was squeezed by a mangle under the conditions of the roll width of 450 mm, the nip width of 20 mm, the nip pressure of 1.45 MPa, and the feed speed of 2 m/min. It was then subjected to drying treatment in a drying furnace at 100°C for 5 minutes, followed by heat-setting at 180°C for 2 minutes. The applied amount of a water-absorbing resin calculated from the increased mass amount of the sheet-like structure (a knitted fabric) after heat-setting was 22.5 g/m$^2$.

Three samples of bioelectrode were prepared by cutting the sheet-like structure thus obtained into a square shape with 15mm $\times$ 15mm. The bioelectrode thus obtained was subjected to calculate the moisture retention index according to the above-described manner with the formula 1 to obtain the index of 0.970. The result was shown in Table 1.

(4) Measurement of Biological Signals

The three samples of bioelectrode thus obtained were pasted on the skin-side surface of a forearm mounting device aligned in a row at an equal interval of 10 mm. The three samples of bioelectrode were connected to a myoelectric potential sensor named "IWS 940 Development Kit-MUSCLE LINK", manufactured by I.W. Technology Firm, Inc., being connected to a positive electrode, a negative electrode and a noise cancel electrode of said sensor respectively in this order through a coated electric wire.

After wetting the samples of bioelectrode with water by spraying tap water by a sprayer, the forearm mounting device was applied to a forearm of a subject so that the samples of bioelectrode were placed in parallel with each other to be in contact with flexor carpi radialis muscle of the subject, and that wearing pressure by the bioelectrode on the skin became 0.5 kPa.

An electromyogram (EMG) of the subject was measured by receiving myoelectric signals while performing bending and stretching exercise of the wrists. The electromyogram thus obtained was shown in Fig.1

The bioelectrode thus obtained was also applied to a subject to evaluate the feeling of wearing caused by contact between the electrode part and the skin by sensory evaluation, and a result of no feeling of discomfort was obtained.

<Example 2>

A bioelectrode was prepared by applying a water-absorbing resin to a sheet-like structure in the same manner as in Example 1, except for using a mixture of 15 parts by mass of a polyester type resin aqueous solution named "SR1801M conc", manufactured by TAKAMATSU OIL & FAT CO., LTD., and 85 parts by mass of water as a water-absorbing resin treatment liquid, and the amount of the water-absorbing resin applied to the sheet-like structure was adjusted to 3.67 g/m$^2$.

The above-described "SR1801M conc" is an emulsion of a polyester resin composition containing an anionic surfactant (an "anionic special polyester resin") which has ability of absorbing and retaining moisture in an amount of 0.6 mass times its own weight.

The bioelectrode thus obtained was subjected to calculate the moisture retention index according to the above-described manner with the formula 1 to obtain the index of 0.823. The result was shown in Table 1. The bioelectrode thus obtained was also used for receiving myoelectric signals in the same manner as in Example 1 to obtain an electromyogram (EMG) which was shown in Fig.2.

The bioelectrode thus obtained was applied to a subject in the same manner as in Example 1 to evaluate the feeling of wearing caused by contact between the electrode part and the skin by sensory evaluation, and a result of no feeling of discomfort was obtained.

<Comparative Example 1>

A bioelectrode was prepared in the same manner as in Example 1, except for not applying the water-absorbing resin to the sheet-like structure.

The bioelectrode thus obtained was subjected to calculate the moisture retention index according to the above-described manner with the formula 1 to obtain the index of 0.125. The result was shown in Table 1. The bioelectrode thus obtained was also used for receiving myoelectric signals in the same manner as in Example 1 to obtain an electromyogram (EMG) which was shown in Fig.3.

The bioelectrode thus obtained was applied to a subject in the same manner as in Example 1 to evaluate the feeling of wearing caused by contact between the electrode part and the skin by sensory evaluation, and a result of a feeling of discomfort caused by wet stickiness was obtained.

<Comparative Example 2>

A bioelectrode was prepared by applying a water-absorbing resin to a sheet-like structure in the same manner as in Example 2, except for using a mixture of 3 parts by mass of a polyester type resin aqueous solution named "SR1801M conc", manufactured by TAKAMATSU OIL & FAT CO., LTD., and 97 parts by mass of water as a water-absorbing resin treatment liquid, and adjusting the amount of the water-absorbing resin applied to the sheet-like structure to 0.94 g/m$^2$.

The bioelectrode thus obtained was subjected to calculate the moisture retention index according to the above-described manner with the formula 1 to obtain the index of 0.186. The result was shown in Table 1. The bioelectrode thus obtained was also used for receiving myoelectric signals in the same manner as in Example 1 to obtain an electromyogram which was shown in Fig.4.

The bioelectrode thus obtained was applied to a subject in the same manner as in Example 1 to evaluate the feeling of wearing caused by contact between the electrode part and the skin by sensory evaluation, and a result of a feeling of discomfort caused by wet stickiness was obtained.

<Evaluation of Biological Signals>

Based on the electromyograms obtained in Examples 1, 2 and Comparative Examples 1,2, evaluation was performed according to the following standard:

○ ; Noise was sufficiently weak for the myoelectric signals and the waveform of myoelectric signals was discriminative.

× ; Noise was strong for the myoelectric signals and the waveform of myoelectric signals could not be discriminated.

(See figures 1-4)

The result was shown in Table 1 with the moisture retention index.

| | Example 1 | Example 2 | Comparative Example 1 | Comparative Example 2 |
|---|---|---|---|---|
| Moisture Retention Index | 0.970 | 0.823 | 0.125 | 0.186 |
| Accuracy of measurement of biological signals | ○ | ○ | × | × |

<Example 3>

[Base Fabric]

A base fabric was prepared by cutting a polyester tricot knitted fabric having a thickness of 300 $\mu$m with cross-knitting of polyurethane elastic yarns, manufactured by SEIREN CO., LTD., wherein the electrode placement region and the input/output region were cut integrally so that the electrode placement region had the size of (a warp direction) 100 mm × (a weft direction) 165 mm and the input/output region had the size of (a warp direction) 60 mm × (a weft direction; the upper side part) 50 mm.

Four restraining parts having the length of 80 mm were formed in the warp direction of the fabric by using a sewing thread. The interval of each restraining parts was 40 mm. The sewing thread was prepared by twisting 3 yarns of a liquid crystal polyester fiber having 280 dtex/48 filaments, named "Zxion Type-vs" manufactured by KB SEIREN, LTD.

[Preparation of Wiring]

A coating film composed of a conductive resin composition was prepared by coating a silver paste named "ELEPASTE TR70901", manufactured by TAIYO INKMFG. CO., LTD., on a release paper having a layer structure of PP/paper/PP with a thickness of 145 $\mu$m, named "WEKR78TPD-H", manufactured by LINTEC Corporation, by means of screen printing using a screen plate made of stainless steel of 100 mesh having a wire diameter of 71 $\mu$m, with an emulsion thickness of 15 $\mu$m. After drying treatment using a hot air circulating drying furnace at 130°C for 30 minutes, a coating film of a conductive resin composition was obtained.

A urethane type hotmelt sheet named "Ecellent SHM-101PUR", manufactured by Sheedom Co., Ltd., was superposed on the coating film of a conductive resin composition thus obtained, to laminate them by heat press using a fully automatic air driven heat transfer press named "HP-4536A-12", manufactured by HASHIMA CO., LTD., at 130°C with pressure of 370 g/cm$^2$ for 30 seconds.

The laminated film thus obtained was cut into a pattern corresponding to the wiring 2 shown in Fig.7 by a laser cutting machine named "LS900", manufactured by Gravotech, Inc., and then a part other than the part of wiring was removed from the release paper to form a wiring pattern with an adhesive layer having a wiring width of 2 mm and a wiring pitch of 40 mm for the electrode placement region.

Then, the adhesive layer of the wiring pattern with an adhesive layer was superposed on the base fabric so that the wiring pattern was provided on the base fabric approximately in the center between the restraining parts in parallel with the sewing thread.

The superposed fabric was then laminated by heat press using a fully automatic air driven heat transfer press named "HP-4536A-12", manufactured by HASHIMA CO., LTD., at 130°C with a pressure of 370 g/cm$^2$ for 30 seconds to form a wiring on the base fabric.

[Preparation of First Insulating Layer]

A polyurethane sheet having a thickness of 100 $\mu$m, named "Higress DUS202" manufactured by Sheedom Co., Ltd., and a urethane type hotmelt sheet having a thickness of 70 $\mu$m, named "Ecellent SHM-101PUR", manufactured by Sheedom Co., Ltd., were superposed on each other, followed by laminating by heat press using a fully automatic air driven heat transfer press named "HP-4536A-12", manufactured by HASHIMA CO., LTD., at 130°C with a pressure of 370 g/cm$^2$ for 30 seconds to form a laminated film.

Subsequently, the laminated film thus obtained was cut into a pattern of an insulating layer by a laser cutting machine named "LS900", manufactured by Gravotech, Inc., and then a part other than the part of the insulating layer pattern was removed to form an insulating layer pattern with an adhesive layer having a line width of 3 mm and a line pitch of 40 mm, which equipped a window having a width of 2 mm and a length of 8 mm opened for connecting with a bioelectrode. The window for connecting with a bioelectrode was provided in one place for each wiring.

The adhesive layer of the insulating layer pattern having an adhesive layer was superposed on the wiring formed on the base fabric, and then they were laminated by heat press using a fully automatic air driven heat transfer press named "HP-4536A-12", manufactured by HASHIMA CO., LTD., at 130°C with a pressure of 370 g/cm$^2$ for 30 seconds to form an insulating layer on the wiring.

[Placement of Bioelectrode]

A knitted fabric of 4-step smooth texture composed of a silver-plated polyester yarn having 33 dtex/6 filaments, manufactured by SEIREN CO., LTD., and a urethane type hotmelt sheet named "Ecellent SHM-101PUR", manufactured by Sheedom Co., Ltd., were superposed on each other, followed by pressing with an iron heated at 110°C for temporary adhesion and cutting into a round shape having a diameter of 10mm to obtain a bioelectrode.

The center of the bioelectrode thus obtained was superposed on the center of the window provided on the insulating layer for connecting with the bioelectrode, and they were laminated by heat press using a fully automatic air driven heat transfer press named "HP-4536A-12", manufactured by HASHIMA CO., LTD., at 130°C with a pressure of 370 g/cm$^2$ for 30 seconds to place the bioelectrode.

[Preparation of Bioelectrode-equipped Apparatus]

With respect to the fabric structure comprising a wiring, an insulating layer and a bioelectrode formed thereon obtained by the above-described steps, both ends of the second extension direction of the electrode placement region were sewn with each other by a sewing machine so as to have the overlapping width of 5 mm, to prepare a bioelectrode-equipped apparatus having an electrode placement region formed in a cylindrical shape and an input/output region protruded in a tongue-like shape on the upper part of the electrode placement region.

The bioelectrode-equipped apparatus thus obtained had a circumferential length of 160 mm. The elongation rate of the first extension direction and that of the second extension direction in the electrode placement region of the apparatus were 2.0% and 45.9% respectively. The second extension direction had an elongation elastic modulus of 89.5%.

[Evaluation of Accuracy of Electrode Positions]

The bioelectrode-equipped apparatus thus obtained was applied to two subjects who had arms having a circumferential length of 170mm and 190mm respectively at a position 50mm away from the ulnar styloid process (a lug portion of the bone on a little finger side of a wrist) in the elbow direction. The position of the bioelectrode in the circumferential length direction at the time of wearing the cylindrical apparatus obtained above was checked. For both two subjects, it was confirmed that the bioelectrode was contacted to an assumed position.

[Evaluation of Difficulty of Disconnection]

The bioelectrode-equipped apparatus obtained above was applied to a subject who had an arm having a circumferential length of 180mm at a position 50mm away from the ulnar styloid process (a lug portion of the bone on a little finger side of a wrist) in the elbow direction. The subject performed wearing and taking off the apparatus 100 times repeatedly. Subsequently, the conducting state of the wiring was measured by a tester named "milliohm HiTESTER 3540", manufactured by HIOKI E.E. CORPORATION, to confirm that the wiring was not disconnected.

<Example 4>

A bioelectrode-equipped apparatus was prepared in the same manner as in Example 3, except for forming a second insulating layer on the same base fabric as of Example 3 and laminating a skin-side fabric before forming a bioelectrode.

[Preparation of Second Insulating Layer]

A polyurethane sheet having a thickness of 100 μm, named "Higress DUS202" manufactured by Sheedom Co., Ltd., and a urethane type hotmelt sheet having a thickness of 70 μm, named "Ecellent SHM-101PUR", manufactured by Sheedom Co., Ltd., were superposed on each other, followed by laminating by heat press using a fully automatic air driven heat transfer press named "HP-4536A-12", manufactured by HASHIMA CO., LTD., at 130°C with a pressure of 370 g/cm$^2$ for 30 seconds to form a laminated film.

The laminated film thus obtained was cut into a pattern of an insulating layer by a laser cutting machine named "LS900", manufactured by Gravotech, Inc., and then a part other than the part of the insulating layer pattern was removed to form an insulating layer pattern with an adhesive layer having a line width of 3 mm and a line pitch of 40 mm.

Then, the insulating layer pattern with an adhesive layer was superposed on the base fabric so that the insulating layer pattern was provided on the base fabric approximately in the center between the restraining parts in parallel with the sewing thread. The superposed fabric was then laminated by heat press using a fully automatic air driven heat transfer press named "HP-4536A-12", manufactured by HASHIMA CO., LTD., at 130°C with a pressure of 370 g/cm$^2$ for 30 seconds to form a second insulating layer pattern on the base fabric.

[Lamination of Skin-side Fabric]

After forming the second insulating layer as described above, the processes described in [formation of a wiring] and [formation of the first insulating layer] of Example 3 were conducted in the same manner as in Example 3. Subsequently, a skin-side fabric was prepared by cutting a polyester knitted fabric having a thickness of 300 $\mu$m with crossknitting of polyurethane elastic yarns, manufactured by SEIREN CO., LTD., to provide a window having a width of 2 mm and a length of 8 mm opened for connecting with a bioelectrode.

A urethane type hotmelt sheet named "Ecellent SHM-101PUR", manufactured by Sheedom Co., Ltd., was cut into a round shape having a diameter of 10 mm and a hole having a width of 2 mm and a length of 8 mm was cut out to prepare a window in the center of the round sheet. The hole and the window of the skin-side fabric were superposed on each other, followed by pressing with an iron heated at 110°C for temporary adhesion of the skin-side fabric with the hotmelt sheet.

Then, the first insulating layer of the base fabric was superposed on the skin-side fabric so that the positions of both windows overlap with each other, and they were laminated by heat press using a fully automatic air driven heat transfer press named "HP-4536A-12", manufactured by HASHIMA CO., LTD., at 130°C with a pressure of 370 g/cm$^2$ for 30 seconds to place the skin-side fabric on the base fabric.

Afterwards, the processes described in [Preparation of Bioelectrode] and [Preparation of Bioelectrode-equipped Apparatus] of Example 3 were conducted in the same manner as in Example 3.

With respect to the bioelectrode-equipped apparatus thus obtained, the elongation rate of the first extension direction and the second extension direction in the electrode placement region were 2.0% and 29.8% respectively. The second extension direction had an elongation elastic modulus of 88.8%.

[Evaluation of Accuracy of Electrode Positions]

The bioelectrode-equipped apparatus thus obtained was subjected to evaluation of accuracy of electrode positions in the same manner as in Example 3, and it was confirmed that the bioelectrode was contacted to an assumed position for both two subjects.

[Evaluation of Difficulty of Disconnection]

The bioelectrode-equipped apparatus obtained above was subjected to evaluation of difficulty of disconnection in the same manner as in Example 3. After wearing and taking off the apparatus 100 times repeatedly, it was confirmed by a tester that the wiring was not disconnected.

<Example 5>

A bioelectrode-equipped apparatus was prepared in the same manner as in Example 3, except for using one bioelectrode obtained in Example 1 as the bioelectrode.

The bioelectrode-equipped apparatus thus obtained was applied to a subject and electromyogram was measured in the same manner as in Example 1. The result was "○" based on the standard indicated in Examples 1, 2 and Comparative Examples 1, 2.

Evaluation of an electrode position was also performed in the same manner as in Example 3 and it was confirmed that the bioelectrode was contacted to an assumed position and that the wiring was not disconnected.

**Industrial Applicability**

The bioelectrode of the present invention is useful for a part of input/output signals to be in contact with a skin, when measuring biometric information such as a heartbeat signal, an electrocardiogram signal, an EEG (electroencephalogram) signal, a myoelectric signal and the like, or when applying electrical stimulation to a living body from an electrode being in contact with an arbitrary position on the skin surface for use in electrical stimulation therapy for motor function recovery of paralyzed patients, a low frequency therapy for improvement of stiffness such as a stiff shoulder by electrical stimulation, and EMS (Electrical Muscle Stimulation) wherein a muscle training effect can be obtained by electrical stimulation.

The bioelectrode of the present invention makes it possible to prevent excess moisture from interposing at a boundary part between the skin and the bioelectrode whereas retaining moisture properly. Therefore, a biological signal having few artifacts can be obtained even during activities. In addition, there is no possibility of a short circuit between adjacent bioelectrodes and of causing discomfort at the time of wearing or measuring biological signals.

The bioelectrode-equipped apparatus of the present invention can be used for a device to be worn on a body (a supporter) by winding around an arm or a leg to fix to a body so that the bioelectrode comes in contact with the skin surface of the body, or by wearing in a cylindrical shape.

Since the apparatus is used so that the second extension direction which exhibits relatively high extensibility is directed along the circumferential direction of an arm or a leg, the apparatus can be applied so that the bioelectrode comes into contact with the predetermined position on the skin surface regardless of the difference in thickness due to individual differences.

Therefore, the bioelectrode-equipped apparatus allows accurate measurement of biological information such as potential difference between electrodes connected to arbitrary positions on a skin surface, impedance, and capacitance. Based on the accurate measurement, it becomes possible to know better biological activities or the state of a subcutaneous tissue. Based on the accurate application of electrical stimulation, it becomes possible to perform a treatment of paralysis or stiffness efficiently or to improve a muscle training effect.

**Claims**

1. A bioelectrode comprising a sheet-like structure containing a conductive fiber and a water-absorbing resin applied to said sheet-like structure, which has a moisture retention index of 0.8 or more.

2. The bioelectrode according to claim 1, wherein said water-absorbing resin is a cross-linked product of a polyacrylic acid and/or a salt thereof.

3. The bioelectrode according to claim 2, wherein said cross-linked product of a polyacrylic acid and/or a salt thereof has a carboxylate equivalent of 150-1500 g/eq.

4. The bioelectrode according to any one of claims 1 to 3, wherein said conductive fiber is a synthetic fiber having a metal film on a surface thereof.

5. The bioelectrode according to any one of claims 1 to 4, wherein said sheet-like structure is a knitted fabric.

6. A bioelectrode-equipped apparatus comprising a fabric structure having an electrode placement region which includes:

   a base fabric having an elastic property,
   a wiring formed on the surface of said base fabric,
   a bioelectrode as a biological signal detection means provided at the terminal end of said wiring, and
   an insulating layer for covering said wiring, wherein said base fabric has, in the electrode placement region,
   a first extension direction which exhibits relatively low extensibility and
   a second extension direction which is different from the first extension direction and which exhibits higher extensibility than the first extension direction, and
   said wiring is formed along the first extension direction.

7. The bioelectrode-equipped apparatus according to claim 6, wherein an elongation rate at a load of 2.1N per 10mm width measured according to a JIS L 1096 D method is 20% or less for the first extension direction in the electrode placement region of said base fabric.

8. The bioelectrode-equipped apparatus according to claim 6, wherein an elongation rate at a load of 2.1N per 10mm width measured according to a JIS L 1096 D method is in the range of 25 to 150% or less for the second extension direction in the electrode placement region of said base fabric.

9. The bioelectrode-equipped apparatus according to claim 6, wherein an elongation elastic modulus measured according to a JIS L 1096 E method at a load of 2.1N per 10mm width with one repeat frequency is 80% or more for the second extension direction in the electrode placement region of said base fabric.

10. The bioelectrode-equipped apparatus according to claim 6, wherein the angle that the first extension direction and the second extension direction form with each other is in the range of 60-90 degrees.

11. The bioelectrode-equipped apparatus according to claim 6, wherein a second insulating layer is provided between the wiring and the base fabric.

**12.** The bioelectrode-equipped apparatus according to claim 6, wherein a skin-side fabric is provided on the surface of the base fabric where the bioelectrode is provided which is the surface to be in contact with a skin in said electrode placement region.

**13.** The bioelectrode-equipped apparatus according to claim 6, wherein said bioelectrode comprises a fabric having a metal film.

**14.** The bioelectrode-equipped apparatus according to claim 6, wherein said bioelectrode is any one of the bioelectrodes according to claims 1 to 5.

**15.** The bioelectrode-equipped apparatus according to claim 6, wherein said wiring comprises a conductive resin composition.

**16.** The bioelectrode-equipped apparatus according to claim 6, wherein a restraining part is provided along the first extension direction.

**17.** The bioelectrode-equipped apparatus according to claim 6, wherein said apparatus is in a cylindrical shape or has a shape that can be fixed by winding around an arm or a leg.

[Fig. 1]

[Fig. 2]

[Fig. 3]

[Fig. 4]

[Fig. 5]

[Fig. 6]

[Fig. 7]

| INTERNATIONAL SEARCH REPORT | International application No. |
|---|---|
| | PCT/JP2020/021068 |

A. CLASSIFICATION OF SUBJECT MATTER
Int.Cl. A61B5/0408(2006.01)i
FI: A61B5/04300W
According to International Patent Classification (IPC) or to both national classification and IPC

B. FIELDS SEARCHED

Minimum documentation searched (classification system followed by classification symbols)
Int.Cl. A61B5/04-5/05

Documentation searched other than minimum documentation to the extent that such documents are included in the fields searched

| | |
|---|---|
| Published examined utility model applications of Japan | 1922–1996 |
| Published unexamined utility model applications of Japan | 1971–2020 |
| Registered utility model specifications of Japan | 1996–2020 |
| Published registered utility model applications of Japan | 1994–2020 |

Electronic data base consulted during the international search (name of data base and, where practicable, search terms used)

C. DOCUMENTS CONSIDERED TO BE RELEVANT

| Category* | Citation of document, with indication, where appropriate, of the relevant passages | Relevant to claim No. |
|---|---|---|
| X<br>Y | JP 2003-299742 A (MINATO MEDICAL SCIENCE CO., LTD.) 21.10.2003 (2003-10-21), paragraphs [0037]-[0041], fig. 1 | 1-5<br>14 |
| Y | JP 2018-201694 A (PANASONIC INTELLECTUAL PROPERTY MANAGEMENT CO., LTD.) 27.12.2018 (2018-12-27), paragraphs [0050]-[0091], fig. 1-4 | 6-17 |
| Y | JP 2017-6348 A (KONICA MINOLTA, INC.) 12.01.2017 (2017-01-12), paragraphs [0031]-[0236], fig. 1-16 | 6-17 |
| Y | WO 2009/041496 A1 (SUMITOMO DAINIPPON PHARMA CO., LTD.) 02.04.2009 (2009-04-02), claims 5-6 | 11 |
| Y | JP 6-296598 A (AGENCY OF INDUSTRIAL SCIENCE AND TECHNOLOGY) 25.10.1994 (1994-10-25), paragraphs [0007]-[0013], fig. 1-4 | 12 |
| Y | JP 2016-7272 A (TANITA CORPORATION) 18.01.2016 (2016-01-18), paragraphs [0016]-[0079], fig. 1-30 | 13 |

☐ Further documents are listed in the continuation of Box C.  ☒ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 12.06.2020 | 11.08.2020 |

| Name and mailing address of the ISA/ | Authorized officer |
|---|---|
| Japan Patent Office<br>3-4-3, Kasumigaseki, Chiyoda-ku,<br>Tokyo 100-8915, Japan | Telephone No. |

Form PCT/ISA/210 (second sheet) (January 2015)

| | | | |
|---|---|---|---|
| JP 2003-299742 A | 21.10.2003 | (Family: none) | |
| JP 2018-201694 A | 27.12.2018 | (Family: none) | |
| JP 2017-6348 A | 12.01.2017 | (Family: none) | |
| WO 2009/041496 A1 | 02.04.2009 | US 2010/0198038 A1 claims 5-6 | |
| JP 6-296598 A | 25.10.1994 | (Family: none) | |
| JP 2016-7272 A | 18.01.2016 | (Family: none) | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2018042719 A
- US 2018078949 A